(19) Europäisches Patentamt / European Patent Office / Office européen des brevets

(11) **EP 4 288 518 B1**

(12) **EUROPÄISCHE PATENTSCHRIFT**

(45) Veröffentlichungstag und Bekanntmachung des Hinweises auf die Patenterteilung:
**05.03.2025 Patentblatt 2025/10**

(21) Anmeldenummer: **21705866.8**

(22) Anmeldetag: **08.02.2021**

(51) Internationale Patentklassifikation (IPC):
*C12Q 1/02* (2006.01)   *C12Q 1/04* (2006.01)
*G01N 15/06* (2024.01)   *G01N 15/1031* (2024.01)
*G01N 33/569* (2006.01)   *G01N 15/10* (2024.01)
*C12M 1/12* (2006.01)   *C12M 1/34* (2006.01)
*C12M 1/36* (2006.01)   *G16H 50/20* (2018.01)
*G01N 15/14* (2024.01)

(52) Gemeinsame Patentklassifikation (CPC):
C12M 37/00; C12M 41/32; C12M 41/36;
C12M 41/48; C12Q 1/02; C12Q 1/04;
G01N 15/0656; G01N 15/1031; G01N 15/1459;
G01N 33/569; G16H 50/20; G01N 15/01;
G01N 2015/1006; G01N 2015/1024;
G01N 2015/1486

(86) Internationale Anmeldenummer:
**PCT/AT2021/060047**

(87) Internationale Veröffentlichungsnummer:
**WO 2022/165541 (11.08.2022 Gazette 2022/32)**

(54) **VORRICHTUNG UND VERFAHREN ZUM REGELN EINES MIKROORGANISMEN-GEHALTS**

DEVICE AND METHOD FOR REGULATING THE CONTENT OF MICROORGANISMS

DISPOSITIF ET PROCÉDÉ POUR RÉGULER LA TENEUR EN MICRO-ORGANISMES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**13.12.2023 Patentblatt 2023/50**

(73) Patentinhaber: **C-Square Bioscience GmbH**
**3430 Tulln an der Donau (AT)**

(72) Erfinder: **KRAETSCHMER, Gerald**
**3443 Sieghartskirchen (AT)**

(74) Vertreter: **Weiser & Voith**
**Patentanwälte Partnerschaft**
**Kopfgasse 7**
**1130 Wien (AT)**

(56) Entgegenhaltungen:
EP-A1- 0 822 261   EP-A1- 1 350 431
EP-A1- 1 938 690   EP-A1- 3 617 691

EP-A2- 0 203 892   EP-B1- 1 133 228
WO-A1-2010/111639   WO-A1-97/48822
WO-A1-99/34013   DE-T2- 69 912 472
US-A1- 2018 259 440

• KLAHRE J ET AL: "Monitoring of biofouling in papermill process waters", WATER RESEARCH, ELSEVIER, AMSTERDAM, NL, vol. 34, no. 14, 1 October 2000 (2000-10-01), pages 3657 - 3665, XP004228647, ISSN: 0043-1354, DOI: 10.1016/ S0043-1354(00)00094-4
• FLEMMING H-C: "biofouling in water systems- cases, causes and coutermeasures", APPLIED MICROBIOLOGY AND BIOTECHNOLOGY, SPRINGER BERLIN HEIDELBERG, BERLIN/ HEIDELBERG, vol. 59, 1 January 2002 (2002-01-01), pages 629 - 640, XP002310946, ISSN: 0175-7598

**Beschreibung**

Die vorliegende Erfindung betrifft eine Vorrichtung zum Regeln eines Gehalts an Mikroorganismen in einer Flüssigkeit, wobei die Vorrichtung eine Messeinheit zum Messen des genannten Mikroorganismen-Gehalts, eine an die Messeinheit angeschlossene Regeleinheit, die dazu ausgebildet ist, zur Erzielung eines vorgegebenen Mikroorganismen-Gehalts eine Dosierung eines Biozids anhand des gemessenen Mikroorganismen-Gehalts zu bestimmen, und eine an die Regeleinheit angeschlossene Zuführeinheit umfasst, welche dazu ausgebildet ist, der Flüssigkeit das Biozid in der von der Regeleinheit bestimmten Dosierung zuzuführen. Die Erfindung betrifft ferner ein Verfahren zum Regeln des Mikroorganismen-Gehalts in der Flüssigkeit.

In einer Vielzahl industrieller Prozesse und Verfahren werden Flüssigkeiten eingesetzt, die während ihres Einsatzes in unerwünschter Weise mit Mikroorganismen belastet sind, so beispielsweise in der chemischen, Baustoff- oder Lebensmittelindustrie beim Reinigen oder Kühlen bzw. bei der Herstellung oder Verarbeitung von z.B. Getränken, Zucker, Stärke, Papier, Zellstoff, Holzwerkstoffen, Kühl- oder Schmierstoffen, Farben, Baustoffen, bei der Fermentation von z.B. Aminosäuren, Antibiotika, Hefe, Zitronensäure, Bioethanol, in der Getränkeabfüllung, der Wasser- und Abwasseraufbereitung, usw.usf. Die Prozesse laufen dabei z.B. in Chargen ("Batches") ab, wobei sich die Flüssigkeit in einem offenen oder geschlossenen Behälter, z.B. einem Prozesstank, befindet und dabei gerührt wird oder weitgehend stationär bleibt. In anderen Prozessen fließt die Flüssigkeit z.B. kontinuierlich durch Kanäle oder Rohre, u.zw. zwischen einem Einlass und einem Auslass oder in einem Kreislauf. In jedem Fall bilden sich in der Flüssigkeit infolge ihrer Zusammensetzung und/oder des industriellen Prozesses und der darin eingesetzten Behälter laufend unerwünschte Mikroorganismen, z.B. Algen, Bakterien, Pilze, insbesondere Hefen, etc., welche die Flüssigkeit kontaminieren und/oder hartnäckige Ablagerungen, sogenannten "Biofilm", oder Korrosion im Behälter verursachen.

Um einen übermäßigen Mikroorganismen-Gehalt in der Flüssigkeit zu verhindern, wird der Flüssigkeit ein Biozid zugesetzt, d.h. ein Mittel mit anti-mikrobieller Wirkung. Im Allgemeinen wird das Biozid kontinuierlich zugeführt; optional erfolgt die Zufuhr des Biozids in einzelnen Dosen. Beispielsweise zeigt die Schrift WO 2010/111639 A1 ein Verfahren und eine Vorrichtung zum Zuführen eines Biozids in einen Tank mithilfe von Pumpen, Ventilen und Durchflussmessern.

Da sich in den genannten industriellen Prozessen einerseits der Gehalt an Mikroorganismen in einer Flüssigkeit und andererseits die Wirkung eines der Flüssigkeit zugeführten Biozids auf den Mikroorganismen-Gehalt nur langsam ändern, ist es heute vielfach üblich, z.B. ein- oder mehrmals täglich Proben der Flüssigkeit zu nehmen, auf ihren Mikroorganismen-Gehalt zu analysieren und dann eine entsprechende Menge an Biozid von Hand zuzuführen oder zumindest die Zuführung, z.B. eine Pumpe, von Hand einzustellen und bis zur nächsten Probenentnahme beizubehalten. Bedingt ist diese Vorgehensweise zumindest unter anderem durch die eingesetzten, oft langsamen Analyseverfahren. Deshalb wird das Biozid im Allgemeinen hoch dosiert, da das meist exponentielle Wachstum der Mikroorganismen bei zu niedriger Dosierung allzu rasch zu einem übermäßigen Mikroorganismen-Gehalt führen kann. Eine Überdosierung hat jedoch negative Auswirkungen z.B. auf die Umwelt infolge von mit Biozid belasteter Flüssigkeit und/oder auf die Prozesskosten infolge des hohen Biozideinsatzes bzw. notwendiger nachträglicher Reinigung der Flüssigkeit von Biozid z.B. bei der biologischen Abwasserbehandlung.

Angesichts neuer, schnellerer Verfahren zum Messen des Mikroorganismen-Gehalts, z.B. nach dem Prinzip der quantitativen PCR ("qPCR"), des Adenosintriphosphat- (ATP-) Tests, des Next Generation Sequencing ("NGS"), der Raman-Spektroskopie oder der Durchflusszytometrie ist nun möglich, die Zufuhr von Biozid in Abhängigkeit von dem gemessenen Mikroorganismen-Gehalt automatisch zu regeln. Beispielsweise ist aus der EP 1 350 431 A1 ein Verfahren bekannt, in welchem ein Analysesystem die in der Flüssigkeit enthaltenen Mikroorganismen ermittelt und ein nachgeschaltetes Biozid-Auswahlsystem anhand der ermittelten Mikroorganismen geeignete Biozide und ihre Konzentration durch Auswahl aus einer Datenbank bestimmt und einem Regler vorgibt, welcher die Biozide in der bestimmten Konzentration der Flüssigkeit geregelt zuführt.

Zwar ermöglicht eine solche automatische Regelung eine raschere Reaktion auf einen steigenden Mikroorganismen-Gehalt bzw. auf eine mangelnde Verringerung des Mikroorganismen-Gehalts infolge der Biozidzufuhr und somit auch einen gezielteren, d.h. auch niedrigeren, Biozideinsatz im Vergleich zu manueller Zufuhr, jedoch muss das Verfahren bzw. die verwendete Datenbank an die jeweils im konkreten industriellen Prozess eingesetzte/n bzw. auftretende/n Flüssigkeit/en und Mikroorganismen angepasst werden, d.h. das Verfahren ist nicht flexibel in seiner Anwendbarkeit. Ferner entsprechen die in der Datenbank hinterlegten Biozide und Konzentrationen jenen bei manueller Zufuhr, sodass auch in diesen Verfahren die zugeführten Biozide in der Regel überdosiert werden, um jedenfalls einen übermäßigen Mikroorganismen-Gehalt zu verhindern.

Die Erfindung setzt sich zum Ziel, eine Vorrichtung und ein Verfahren zum Regeln des Gehalts an Mikroorganismen in einer Flüssigkeit zu schaffen, welche für unterschiedliche industrielle Prozesse, unterschiedliche Flüssigkeiten und unterschiedliche Mikroorganismen einfach, flexibel und zuverlässig einsetzbar sind und dabei eine Überdosierung von Biozid vermeiden.

Dieses Ziel wird gemäß einem ersten Aspekt der Erfindung mit einer Vorrichtung der einleitend genannten Art erreicht, die sich durch eine an die Messeinheit und die Regeleinheit angeschlossene Recheneinheit auszeichnet, wobei die Regeleinheit dazu ausgebildet ist, die Dosierung unter Verwendung eines Modells mit zumindest einem Parameter des Zusammenhangs zwischen einer Menge an in einem Zeitintervall zugeführtem Biozid und der dadurch in diesem Zeitintervall verursachten Änderung des Mikroorganismen-Gehalts zu bestimmen, und wobei die Recheneinheit dazu ausgebildet ist, aus einer Aufzeichnung des von der Messeinheit über zumindest ein vergangenes Zeitintervall gemessenen Mikroorganismen-Gehalts und der Menge an in diesem Zeitintervall zugeführtem Biozid den zumindest einen Parameter zur Verwendung durch die Regeleinheit in zumindest einem späteren Zeitintervall zu berechnen.

Das Modell ist ein kinetisches Modell der Reaktion der Flüssigkeit, d.h. der Änderung ihres Mikroorganismen-Gehalts, auf die Menge an zugeführtem Biozid; damit sind Reaktionen aller mit Mikroorganismen belasteten Flüssigkeiten auf Zufuhr von Biozid modellierbar. Mithilfe der Recheneinheit wird durch einfache Berechnung des oder der Parameter/s das Modell an verschiedene Flüssigkeiten und vor allem an die dynamischen Veränderungen einer Flüssigkeit mit den darin enthaltenen Mikroorganismen bzw. ihrer Reaktion auf die Biozidzufuhr angepasst, also nachgeführt. Durch die Verwendung dieses parametrierten Modells der Reaktion passt sich zugleich die Vorrichtung bzw. ihre Regeleinheit an den jeweiligen Prozess und die dabei eingesetzte bzw. auftretende Flüssigkeit und die darin enthaltenen Mikroorganismen an und die Regelung wird nachgeführt. Die Vorrichtung der Erfindung ist damit besonders einfach und flexibel in der Anwendung und regelt den Mikroorganismen-Gehalt der Flüssigkeit besonders effektiv, wodurch erhebliche Mengen an Biozid eingespart werden.

In einer ersten Variante der Erfindung ist das genannte Modell durch die Gleichung

$$\frac{dX}{dt} = -kB^n X$$

gegeben, mit:

$\dfrac{dX}{dt}$  Änderung des Mikroorganismen-Gehalts in einem Zeit-intervall,

k  Parameter des Modells,

B  Menge an in diesem Zeitintervall zugeführtem Biozid,

n  Verdünnungskoeffizient des Biozids und

X  gemessener Mikroorganismen-Gehalt.

Dies stellt ein besonders einfaches und universell einsetzbares Modell dar, dessen Parameter mit geringem Aufwand bestimmt werden kann/können. Der Verdünnungskoeffizient des Biozids kann darin entweder vorbekannt sein, z.B. wenn das eingesetzte Biozid und die Flüssigkeit bzw. die darin enthaltenen Mikroorganismen hinreichend genau bekannt sind, oder er wird von der Recheneinheit als weiterer Parameter des Modells berechnet; solch weitere Parameter berechnet die Recheneinheit z.B. aus der Aufzeichnung über zumindest entsprechend viele weitere vergangene Zeitintervalle.

Dasselbe gilt für eine zweite Variante der Erfindung, gemäß welcher das genannte Modell durch die Gleichung

$$\frac{dX}{dt} = -k_1 B_0^n e^{-k_2 nt} X$$

gegeben ist, mit:

$\dfrac{dX}{dt}$  Änderung des Mikroorganismen-Gehalts in einem Zeitintervall,

$k_1, k_2$  Parameter des Modells,

$B_0$  Menge an in diesem Zeitintervall zugeführtem Biozid zum Zeitpunkt seiner Zufuhr,

n  Verdünnungskoeffizient des Biozids und

X  gemessener Mikroorganismen-Gehalt.

Dieses erweiterte kinetische Modell berücksichtigt die mögliche Eigenschaft eines Biozids, nach seiner Zufuhr zur Flüssigkeit über die Zeit abgebaut bzw. inaktiviert zu werden.

Die Messeinheit kann den Gehalt an Mikroorganismen in der Flüssigkeit auf verschiedene Weise messen, beispielsweise nach dem Prinzip der quantitativen PCR ("qPCR"), des Adenosintriphosphat- (ATP-) Tests, des Next Generation Sequencing ("NGS") oder der Raman-Spektroskopie. Besonders vorteilhaft ist, wenn die Messeinheit ein Durchflusszytometer ist, welches dazu ausgebildet ist, den Gehalt an Mikroorganismen einer vorgegebenen Art zu messen. Durchflusszytometer erlauben eine parallele Auswertung mehrerer Messkanäle mit hoher Geschwindigkeit, d.h. auch in Echtzeit, und dabei eine Unterscheidung der Mikroorganismen anhand unterschiedlicher Fluoreszenz, Struktur, Farbe, elektrischer Eigenschaften etc. unter optionaler Zuhilfenahme verschiedener Fluoreszenzfarbstoffe. Im Durchflusszytometer werden die Mikroorganismen in rascher Aufeinanderfolge einzeln entlang einer hohen elektrischen Spannung und/oder durch einen Laserstrahl geführt und ihre jeweilige spezifische Reaktion auf das elektrische Feld (z.B. Ablenkung) und/oder den Laserstrahl (z.B. Reflexion, Farbe, Fluoreszenz etc.) erfasst und ausgewertet. Meist werden dabei multi-dimensionale Datenfelder erzeugt, in welchen die erfassten Mikroorganismen z.B. durch (multi-dimensionale) Clusterbildung automatisch klassifiziert und dadurch unterschieden werden. Auf diese Weise können die Mikroorganismen der vorgegebenen Art aufgefunden und selektiv gezählt werden. Zum Klassifizieren kann optional ein lernendes System eingesetzt werden, wie aus der EP 3 617 691 A1 bekannt ist.

Besonders günstig ist, wenn die Recheneinheit dazu ausgebildet ist, den zumindest einen Parameter nach jedem Zeitintervall einer Folge von Zeitintervallen aus der Aufzeichnung des von der Messeinheit über zumindest ein unmittelbar vorangegangenes Zeitintervall gemessenen Mikroorganismen-Gehalts und der Menge an in diesem zumindest einen unmittelbar vorangegangenen Zeitintervall zugeführtem Biozid zu berechnen. Der zumindest eine Parameter des Modells wird dadurch laufend nachgeführt, sodass die Regelung an eine allfällige Veränderung der Eigenschaft der mit den Mikroorganismen belasteten Flüssigkeit jeweils unmittelbar angepasst wird und dadurch besonders gute Wirkung erzielt. Die Zeitintervalle der Folge können dabei an die jeweiligen Erfordernisse angepasst werden und hängen insbesondere davon ab, wie schnell die Flüssigkeit ihre Eigenschaften verändert.

In einer bevorzugten Ausführungsform der Vorrichtung ist die Zuführeinheit dazu ausgebildet, das Biozid gemäß einer vorgegebenen Zusammensetzung aus einer oder mehreren Komponenten zu wählen, wobei die Vorrichtung ferner eine an die Zuführeinheit angeschlossene Steuereinheit umfasst, welche dazu ausgebildet ist, der Zuführeinheit die Zusammensetzung vorzugeben. Dies ermöglicht eine über die Dosierung hinausgehende Optimierung, um jeweils ein für den gemessenen Gehalt und die gemessene/n Art/en an Mikroorganismen besonders effektives Biozid aus einer oder mehreren Komponenten einzusetzen.

Besonders günstig ist dabei, wenn die Vorrichtung zumindest einen Sensor zum Messen zumindest einer der Messgrößen pH-Wert, Temperatur, Druck und Leitfähigkeit der Flüssigkeit umfasst, wobei die Steuereinheit an den zumindest einen Sensor angeschlossen und dazu ausgebildet ist, die Zusammensetzung in Abhängigkeit von zumindest dieser/diesen Messgröße/n vorzugeben. Die Optimierung des Biozids erfolgt dadurch anhand von konkret gemessenen Flüssigkeitseigenschaften, welche z.B. erfahrungsgemäß Einfluss auf den Gehalt an Mikroorganismen der Flüssigkeit haben. Beispielsweise kann das Biozid dabei einerseits neben zumindest einer antimikrobiellen Komponente zumindest eine Komponente anderer Wirkung enthalten, welche z.B. den pH-Wert oder die Leitfähigkeit der Flüssigkeit beeinflusst, sodass die antimikrobiellen Komponente des Biozids besonders wirksam ist; anderseits können verschiedene antimikrobielle Komponenten kombiniert werden. Die Zusammensetzung kann nach der bzw. den gemessenen Messgröße/n bzw. nach anderen Aspekten, z.B. dem Mikroorganismen-Gehalt in der Flüssigkeit, der Umweltfreundlichkeit der Komponenten und/oder ihrer Kosten, vorgegeben und gewählt werden. Das genannte Wählen umfasst bei zwei oder mehr Komponenten auch deren Vermischen oder das Lösen einer Komponente in der anderen; oder die gewählten Komponenten werden der Flüssigkeit separat zugeführt.

Besonders vorteilhaft ist, wenn die Steuereinheit dazu ausgebildet ist, in regelmäßigen Zeitabständen der Zuführeinheit eine Zusammensetzung vorzugeben. Falls keine Änderung der Zusammensetzung erforderlich ist, kann dabei die Steuereinheit auch mehrmals aufeinanderfolgend dieselbe Zusammensetzung vorgeben. Ferner kann zumindest gelegentlich eine zufällige Zusammensetzung des Biozids vorgegeben werden, wodurch die Steuereinheit die Wirksamkeit verschiedener Zusammensetzungen testen und - z.B. wenn die Steuereinheit ein selbst-lernendes System ist - erlernen kann, sodass sie später anhand des Gelernten eine optimale Zusammensetzung vorgeben kann.

In einem zweiten Aspekt schafft die Erfindung ein Verfahren zum Regeln des Gehalts an Mikroorganismen in einer Flüssigkeit, umfassend
in einem Regelprozess:

Messen des genannten Mikroorganismen-Gehalts mithilfe einer Messeinheit,
Bestimmen einer Dosierung eines Biozids in Hinblick auf die Erzielung eines vorgegebenen Mikroorganismen-Gehalts anhand des gemessenen Mikroorganismen-Gehalts und eines Modells mit zumindest einem Parameter des Zusammenhangs zwischen einer Menge an in einem Zeitintervall zugeführtem Biozid und der dadurch in diesem Zeitintervall verursachten Änderung des Mikroorganismen-Gehalts, und
Zuführen des Biozids in der bestimmten Dosierung zu der Flüssigkeit; und
in einem zum Regelprozess parallelen oder intermittierenden Nachführprozess:

Berechnen des zumindest einen Parameters aus einer Aufzeichnung des von der Messeinheit über zumindest ein vergangenes Zeitintervall gemessenen Mikroorganismen-Gehalts und der Menge an in diesem Zeitintervall zugeführtem Biozid zur Verwendung in dem Regelprozess in zumindest einem späteren Zeitintervall.
Bezüglich der Vorteile des Verfahrens und weiterer Varianten wird auf die vorangegangenen Ausführungen zur Vorrichtung verwiesen.

Die Erfindung wird nachfolgend anhand von in den beigeschlossenen Zeichnungen dargestellten Ausführungsbeispielen näher erläutert. In den Zeichnungen zeigt:

Fig. 1 eine erfindungsgemäße Vorrichtung zum Regeln eines Gehalts an Mikroorganismen in einer Flüssigkeit in einem Blockschaltbild;

Fig. 2 ein Diagramm der Fluoreszenzeigenschaften von mit einer Messeinheit der Vorrichtung von Fig.1 erfassten Mikroorganismen;

Fig. 3 ein Diagramm der Zufuhr an Biozid und des Mikroorganismen-Gehalts in der Flüssigkeit über der Zeit bei Verwendung der Vorrichtung von Fig. 1; und

Fig. 4 ein von der Vorrichtung von Fig. 1 ausgeführtes beispielhaftes Verfahren zum Regeln des Mikroorganismen-Gehalts in der Flüssigkeit in einem Flussdiagramm.

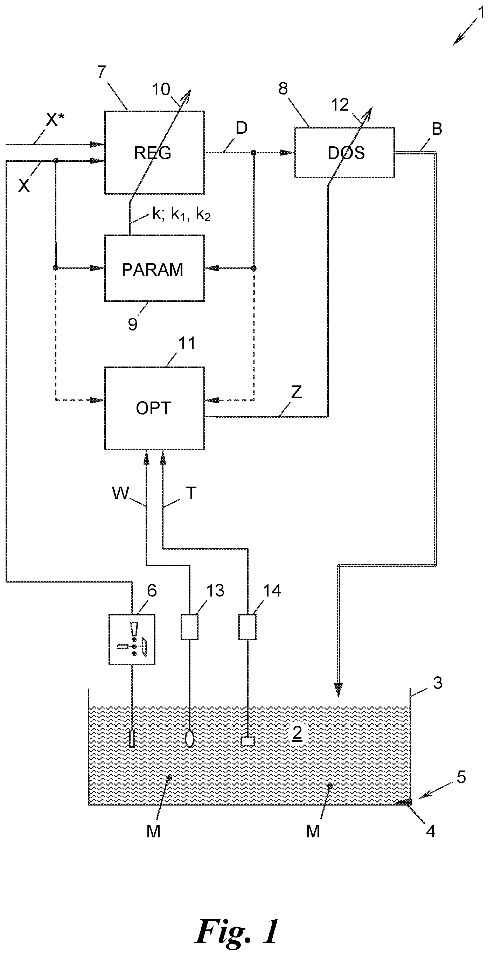

Eine Vorrichtung 1 gemäß Fig. 1 regelt einen Gehalt X an Mikroorganismen M in einer Flüssigkeit 2, um einen vorgegebenen Mikroorganismen-Gehalt $X^*$ zu erzielen. Bei den Mikroorganismen M handelt es sich z.B. um Algen, Bakterien, Pilze, insbesondere Hefen, etc. Die Flüssigkeit 2 wird z.B. in einem industriellen Prozess zur Herstellung, Verarbeitung, Kühlung, Reinigung od.dgl. eines Objekts oder einer Substanz verwendet oder ist - z.B. als Getränk in einer Abfüllanlage - der eigentliche Prozessgegenstand. Der industrielle Prozess kann dabei ein Chargen- bzw. Batch-Prozess sein, in welchem die Flüssigkeit 2 batchweise anfällt bzw. eingesetzt wird, oder es handelt sich um einen kontinuierlichen Prozess, bei welchem auch die Flüssigkeit 2 kontinuierlich eingesetzt wird.

Die Flüssigkeit 2 befindet sich in einem Behälter (hier: einem offenen Tank) 3, und Mikroorganismen M haben - in diesem Beispiel - infolge ihrer Vermehrung eine unerwünschte Ablagerung 4 in einer Ecke 5 des Tanks 3 gebildet. Alternativ vermehren sich die Mikroorganismen M in der Flüssigkeit, ohne eine Ablagerung 4 zu bilden, und kontaminieren die Flüssigkeit 2 oder sind aus anderen Gründen unerwünscht.

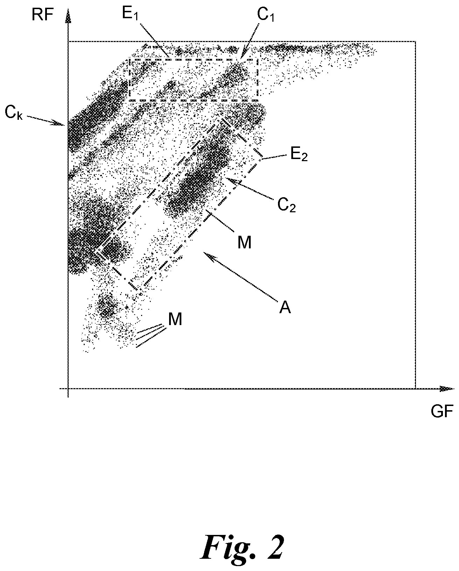

Eine Messeinheit 6 misst den genannten Mikroorganismen-Gehalt X in der Flüssigkeit 2. Der Gehalt X gibt die Anzahl an Mikroorganismen M pro Bezugsgröße der Flüssigkeit 2 an, d.h. pro Volumen (z.B. Milliliter), pro Masse (z.B. Gramm) oder pro Menge (z.B. Mol) der Flüssigkeit 2. Zum Messen des Mikroorganismen-Gehalts X kann die Messeinheit 6 unterschiedliche Verfahren anwenden, z.B. eine quantitative PCR ("qPCR"), einen Adenosintriphosphat- (ATP-) Test, ein Next Generation Sequencing ("NGS") Messverfahren oder eine Raman-Spektroskopie. Im dargestellten Fall ist die Messeinheit 6 ein Durchflusszytometer, welches den Gehalt X an Mikroorganismen M einer vorgegebenen Art A misst, wie im Folgenden anhand des Beispiels der Fig. 2 näher erläutert wird.

In einem Durchflusszytometer werden Partikel in rascher Aufeinanderfolge einzeln an einer hohen elektrischen Spannung und/oder einem Laserstrahl vorbeigeführt und ihre jeweilige spezifische Reaktion auf das elektrische Feld (z.B. Ablenkung) und/oder den Laserstrahl (z.B. Reflexion, Farbe, Fluoreszenz etc.) erfasst und ausgewertet. Durchflusszytometer erlauben eine parallele Auswertung mehrerer Messkanäle mit hoher Geschwindigkeit, d.h. auch in Echtzeit, und unterscheiden Partikel nach ihren Eigenschaften bzw. Reaktionen; optional werden dabei Hilfsstoffe, z.B. Fluoreszenzfarbstoffe, eingesetzt. Dabei werden meist multi-dimensionale Datenfelder erzeugt, in welchen die erfassten Partikel z.B. durch (multidimensionale) Clusterbildung automatisch klassifiziert und dann optional z.B. nach Klasse gezählt werden können. Dadurch kann optional die Art A von Mikroorganismen M vorgegeben werden, deren Gehalt X in der Flüssigkeit 2 (selektiv) gemessen werden soll.

Das beispielhafte zweidimensionale Diagramm der Fig. 2 zeigt Mikroorganismen M, welche mittels Durchflusszytometrie detektiert, nach ihren Eigenschaften (hier zwei Eigenschaften, nämlich Rot- und Grün-Fluoreszenz) unterschieden und aufgezeichnet wurden. In dem Diagramm ist horizontal die Grünfluoreszenz und vertikal die Rotfluoreszenz (hier: jeweils im logarithmischen Maßstab) aufgetragen. Weitere physikalische oder chemische Eigenschaften (z.B. andere Fluoreszenzen etc.) können auf weiteren Messkanälen erfasst und z.B. in weiteren Dimensionen ausgewertet werden. Mikroorganismen M mit ähnlichen Eigenschaften (hier: in diesen beiden Wellenlängenbereichen) bilden in dem Diagramm jeweils Cluster $C_1$, $C_2$, ..., allgemein $C_k$. Anhand bekannter Cluster $C_k$ oder solcher, die mithilfe einer lernenden Cluster-Auswertung ermittelt wurden, können verschiedene Mikroorganismen M jeweils voneinander unterschieden werden; beispielsweise sind im Diagramm der Fig. 2 Algen durch ein strichliertes Rechteck $E_1$ gekennzeichnet und von Bakterien (strichpunktiertes Rechteck $E_2$) unterschieden. Als die genannte Art A können deshalb z.B. entweder Algen oder (hier) Bakterien etc. vorgegeben werden. Alternativ können als die genannte Art A bestimmte Algen, z.B. Blaualgen, oder bestimmte Bakterien, z.B. Milchsäurebakterien, etc. oder überhaupt eine Art A von Mikroorganismen M mit besonderen Eigenschaften vorgegeben werden, soweit diese in der Durchfusszytometrie unterscheidbar sind, z.B. könnte durch die vorgegebene Art A differenziert werden, ob ein Mikroorganismus M viabel ist oder nicht und nur die viablen Mikroorganismen M als Art A vorgegeben sein. Schließlich können als Art A verschiedene Mikroorganismen M, z.B. Algen und Bakterien, gemeinsam vorgegeben werden.

Sind im Fall der Fig. 2 beispielsweise die genannten Bakterien als die Art A vorgegeben, misst die Messeinheit 6 deren Gehalt X in der Flüssigkeit 2, d.h. andere Mikroorganismen M bzw. Partikel, die gar keine Mikroorganismen M sind, werden in diesem Beispiel von der Messeinheit 6 beim Messen des Mikroorganismen-Gehalts X nicht weiter berücksichtigt. Aus Diagrammen aufeinanderfolgender Zeitintervalle Δt kann eine Veränderung der Mikroorganismen M, insbesondere ihres Gehalts X, in der Flüssigkeit 2 über der Zeit ermittelt werden.

Es versteht sich, dass die Messeinheit 6 den Mikroorganismen-Gehalt X im Allgemeinen anhand einer Probe von aus dem Behälter 3 entnommener Flüssigkeit 2 misst.

Zurückkommend auf die Vorrichtung 1 der Fig. 1 ist an die Messeinheit 6 eine Regeleinheit 7 angeschlossen, sodass sie Zugriff auf den von der Messeinheit 6 jeweils gemessenen Mikroorganismen-Gehalt X hat. Die Regeleinheit 6 hat z.B. einen Proportional-Integral- (PI-), einen Proportional-Integral-Differential- (PID-) Regler od.dgl. zum Regeln des Mikroorganismen-Gehalts X. Dazu bestimmt die Regeleinheit 7 eine Dosierung D eines Biozids B, welches der Flüssigkeit 2 zugesetzt werden soll, um den vorgegebenen Mikroorganismen-Gehalt X* zu erzielen. Der vorgegebene Mikroorganismen-Gehalt X* kann dabei einerseits konstant oder andererseits zeitlich veränderlich vorgegeben werden. Die zeitliche Veränderung des vorgegebenen Mikroorganismen-Gehalts X* beruht z.B. auf einer Anpassung an einen vorhergeplanten Verlauf und/oder einen jeweils gemessenen Zustand des industriellen Prozesses. Beispielsweise ist im Rahmen eines sukzessive Flüssigkeit 2 zuführenden sogenannten "Fed-Batch"-Prozesses - z.B. in einem Fermentationsverfahren - nicht nur die Menge der Flüssigkeit 2 vom Prozessfortschritt abhängig, d.h. zeitlich veränderlich, sondern meist auch der vorgegebene Mikroorganismen-Gehalt X*; insbesondere kann dabei in einem anfänglichen Prozessabschnitt die Flüssigkeitsmenge gering und ein z.B. hoher vorgegebener Mikroorganismen-Gehalt X* gewünscht sein, wogegen in einem späteren Prozessabschnitt die Flüssigkeitsmenge größer und der vorgegebene Mikroorganismen-Gehalt X* zu reduzieren ist.

Die Vorrichtung 1 umfasst ferner eine Zuführeinheit 8 mit einem oder mehreren Reservoirs, aus welchen sie der Flüssigkeit 2, z.B. mithilfe einer steuerbaren Pumpe oder eines Regelventils, Biozid B zuführt. Die Zuführeinheit 8 ist an die Regeleinheit 7 angeschlossen, um der Flüssigkeit 2 das Biozid B in der von der Regeleinheit 7 bestimmten Dosierung D zuzuführen.

Zum Bestimmen der Dosierung D verwendet die Regeleinheit 7 ein Modell mit zumindest einem Parameter k. Das Modell bildet einen Zusammenhang zwischen einer Menge P von in einem Zeitintervall Δt zugeführtem Biozid B und der dadurch in diesem Zeitintervall Δt verursachten Änderung des Mikroorganismen-Gehalts X ab, d.h. es ist ein kinetisches Modell der Reaktion der Flüssigkeit 2 und somit ihres Mikroorganismen-Gehalts X auf das zugeführte Biozid B. Es versteht sich, dass die Menge P von im Zeitintervall Δt zugeführtem Biozid B mit der Dosierung D des der Flüssigkeit 2 zuzuführenden Biozid B zusammenfällt, wenn diese für dasselbe Zeitintervall Δt bestimmt wurde. Die Regeleinheit 7 verwendet das Modell, um eine oder mehrere Zeitkonstanten und/oder Verstärkungen ("gains") ihres Reglers an die Reaktion der Flüssigkeit 2, d.h. die Änderung ihres Mikroorganismen-Gehalts X, auf Biozidzufuhr anzupassen.

Das Modell kann verschiedener Art sein, insbesondere ein Modell, welches eine lineare, quadratische oder exponentielle Abnahme des Mikroorganismen-Gehalt X bei Biozidzufuhr annimmt. In einer ersten Variante der Erfindung ist das Modell durch die folgende Gleichung 1 gegeben:

$$\frac{dX}{dt} = -kB^n X \qquad\qquad (1)$$

mit:

$\dfrac{dX}{dt}$ Änderung des Mikroorganismen-Gehalts X in einem Zeit-intervall Δt (hier: dem "infinitesimalen" Zeitintervall dt),

k Parameter des Modells betreffend eine Deaktivierung der Mikroorganismen M,

B Menge P an in diesem Zeitintervall Δt zugeführtem Biozid B,

n Verdünnungskoeffizient des Biozids B und

X gemessener Mikroorganismen-Gehalt X.

In einer zweiten Variante der Erfindung ist das Modell durch die folgende Gleichung 2 gegeben:

$$\frac{dX}{dt} = -k_1 B_0^n e^{-k_2 nt} X \qquad\qquad (2)$$

mit: $\dfrac{dX}{dt}$ Änderung des Mikroorganismen-Gehalts X in einem Zeit-intervall Δt (hier: dem "infinitesimalen" Zeitintervall dt),

$k_1$ Parameter des Modells betreffend eine Deaktivierung der Mikroorganismen M,

$k_2$ Parameter des Modells betreffend eine Inaktivierung des Biozids B nach seiner Zufuhr zur Flüssigkeit 2,

$B_0$ Menge P an in diesem Zeitintervall Δt zugeführtem Biozid B zum Zeitpunkt seiner Zufuhr,

n    Verdünnungskoeffizient des Biozids B und

X    gemessener Mikroorganismen-Gehalt X.

Das erweiterte Modell der Gleichung (2) ermöglicht durch den Parameter $k_2$, eine Inaktivierung des Biozids B nach seiner Zufuhr zu der Flüssigkeit 2 zu berücksichtigen.

In beiden vorgenannten Modellen ist der Verdünnungskoeffizient n des Biozids B entweder vorbekannt, wenn das eingesetzte Biozid B und die Flüssigkeit 2 und die darin enthaltenen Mikroorganismen M bekannt sind; andernfalls ist der Verdünnungskoeffizient n ein weiterer Parameter des Modells, sodass das durch die Gleichung (1) gegebene Modell zwei Parameter k und n und das durch die Gleichung (2) gegebene Modell drei Parameter $k_1$, $k_2$ und n hat.

Soll das Modell auch einen Einfluss allfälliger Nebenreaktionen des industriellen Prozesses auf den Zusammenhang zwischen der Menge P an zugeführtem Biozid B und der dadurch verursachten Änderung des Mikroorganismen-Gehalts X abbilden, kann das Modell optional z.B. durch ein System miteinander gekoppelter Differentialgleichungen gegeben sein, welche jeweils den Einfluss des industriellen Prozesses selbst bzw. jenen seiner Nebenreaktionen beschreiben.

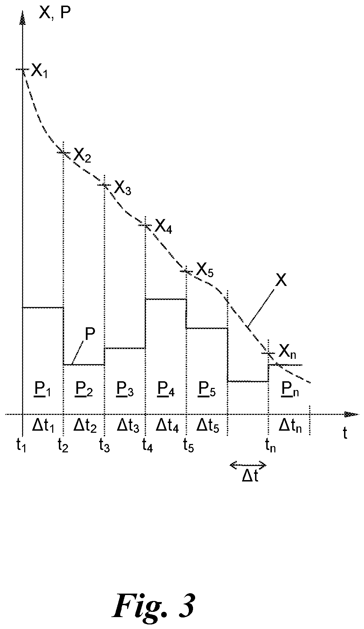

Um das Modell bzw. seinen zumindest einen Parameter k, $k_1$, $k_2$, n und damit die Regeleinheit 7 an die Reaktion der Flüssigkeit 2 auf Biozidzufuhr anzupassen und auch bei dynamischen Änderungen dieser Reaktion nachzuführen, hat die Vorrichtung 1 eine Recheneinheit 9, die an die Messeinheit 6 und die Regeleinheit 7 angeschlossen ist und die Regeleinheit 7 über einen Steuerpfad 10 nachführt. Die Recheneinheit 9 berechnet den bzw. die Parameter k bzw. $k_1$, $k_2$ etc. (und erforderlichenfalls als weiteren Parameter den Verdünnungskoeffizienten n) des jeweiligen Modells und nutzt dazu eine Aufzeichnung des von der Messeinheit 6 über zumindest ein vergangenes Zeitintervall $\Delta t$ gemessenen Mikroorganismen-Gehalts X und eine Menge P an Biozid B, welches in diesem Zeitintervall $\Delta t$ der Flüssigkeit 2 zugeführt wurde, wie im Folgenden anhand des Beispiels der Fig. 3, das ein Modell mit einem Parameter k betrifft, näher erläutert wird. Es versteht sich, dass in dem Fall eines Modells mit zwei oder mehr Parametern $k_1$, $k_2$, n, ... die Recheneinheit 9 diese aus Aufzeichnungen über zumindest entsprechend viele vorangegangene Zeitintervalle $\Delta t$ berechnen kann.

Fig. 3 zeigt eine Folge von Zeitintervallen $\Delta t_1$, $\Delta t_2$, ..., allgemein $\Delta t_n$, welche jeweils zwischen zwei Zeitpunkten $t_1$, $t_2$, ..., allgemein $t_n$, liegen. Zumindest in jedem Zeitpunkt $t_n$ misst die Messeinheit 6 jeweils den Mikroorganismen-Gehalt $X_1$, $X_2$, ..., allgemein $X_n$ (hier: ein kontinuierlich gemessener Verlauf des Mikroorganismen-Gehalts X). In jedem Zeitintervall $\Delta t_n$ der Folge wird jeweils eine Menge $P_1$, $P_2$, ..., allgemein $P_n$, an Biozid B zugeführt (hier: kontinuierlich während jedes Zeitintervalls $\Delta t_n$). Wird beispielsweise in einem Zeitintervall $\Delta t_4$ zwischen den Zeitpunkten $t_4$ und $t_5$ der Flüssigkeit 2 eine Menge $P_4$ an Biozid B zugeführt, verursacht dies eine Änderung des Mikroorganismen-Gehalts X von einem Wert $X_4$ im Zeitpunkt $t_4$ auf einen Wert $X_5$ im Zeitpunkt $t_5$.

Der genannte Parameter k des Modells kann nach einem beliebigen oder nach jedem Zeitintervall $\Delta t_n$ der Folge jeweils von der Recheneinheit 9 berechnet werden. Soll beispielsweise der Parameter k für das Zeitintervall $\Delta t_4$ berechnet werden, entnimmt die Recheneinheit 9 nach Ablauf dieses Zeitintervalls $\Delta t_4$ aus der Aufzeichnung (Fig. 3) den von der Messeinheit 6 über dieses Zeitintervall $\Delta t_4$ gemessenen Mikroorganismen-Gehalt $X_4$ und die Menge $P_4$ an in diesem Zeitintervall $\Delta t_4$ zugeführtem Biozid B. Der Parameter k wird anhand dieser Menge $P_4$ und der dadurch verursachten Änderung $\Delta X$ (bzw. infinitesimal: dX) des Mikroorganismen-Gehalts X (z.B. gemäß $\Delta X = X_5 - X_4$) berechnet und zur Verwendung durch die Regeleinheit 7 in einem oder mehreren späteren Zeitintervallen $\Delta t_n$ (hier: z.B. $\Delta t_5$ und/oder $\Delta t_6$) an diese übermittelt.

Optional kann die Recheneinheit 9 eine Abgleicheinrichtung enthalten, welche den jeweils berechneten Parameter k vor der Übergabe an die Regeleinheit 7 mit für vorangegangene Zeitintervalle $\Delta t$ berechneten Werten des Parameters k durch - z.B. gewichtete - Mittelwertbildung mittelt bzw. abgleicht. Die Abgleicheinrichtung ist z.B. ein zusätzlicher Rechenbaustein der Recheneinheit 9 oder ein neuronales Netz, insbesondere ein Long Short-Term Memory (LSTM), das beim Abgleichen optional zusätzlich in vorangegangenen Zeitintervallen $\Delta t$ verursachte Änderungen des Mikroorganismen-Gehalts X berücksichtigt. Die Regeleinheit 7 verwendet in diesem Fall den abgeglichenen Parameter k des Modells zur Anpassung ihres Reglers, wodurch vergangene Parameteränderungen Berücksichtigung finden und einer sprunghaften Anpassung des Reglers vorgebeugt wird.

Zurückkommend auf Fig. 1 ist die Zuführeinheit 8 in einer optionalen Ausführungsform dazu ausgebildet, das Biozid B aus einer oder mehreren Komponenten zu wählen, welche die Zuführeinheit 8 jeweils in einem Reservoir bereithält, um eine vorgegebene Zusammensetzung Z des Biozids B zu erzielen. In dieser Ausführungsform umfasst die Vorrichtung eine Steuereinheit 11, welche an die Zuführeinheit 8 angeschlossen ist und der Zuführeinheit 8 die genannte Zusammensetzung Z vorgibt. Die Steuereinheit 11 greift somit in das Zuführen des Biozids B ein, u.zw. indem über einen Steuerpfad 12 die Zusammensetzung Z des Biozids B vorgegeben bzw. verändert wird.

Die Zusammensetzung Z kann dabei optional - zumindest gelegentlich - zufällig vorgegeben werden; alternativ dazu werden die Komponenten der Zusammensetzung Z z.B. heuristisch gewählt, wie weiter unten näher erläutert wird. Optional umfasst die Vorrichtung 1 dazu einen oder mehrere (im Beispiel von Fig. 1: zwei) Sensoren 13, 14, welche zumindest eine Messgröße (meist jeweils eine Messgröße pro Sensor 13, 14) messen, z.B. den pH-Wert W, die Temperatur T, den Druck und/oder die Leitfähigkeit der Flüssigkeit 2. Im Beispiel der Fig. 1 misst ein Sensor 13 der genannten Sensoren 13, 14 den pH-Wert W und der andere Sensor 14 die Temperatur T der Flüssigkeit 2. Die Steuereinheit 11 gibt die Zusammensetzung Z in diesem Fall in Abhängigkeit von zumindest diesen Messgrößen W, T vor.

Beim Vorgeben der Zusammensetzung Z des Biozids B ist die Steuereinheit 11 ferner optional an die Messeinheit 6 angeschlossen, um die Zusammensetzung Z auch in Abhängigkeit vom Mikroorganismen-Gehalt X vorzugeben. Ferner ist die Steuereinheit 11 optional an die Regeleinheit 7 angeschlossen, um die von dieser bestimmte Dosierung D zu erhalten, sodass die Steuereinheit 11 die Zusammensetzung Z auch in Abhängigkeit von der Dosierung D bzw. der Menge P an im Zeitintervall $\Delta t$ zugeführtem Biozid B, von der oder den daraus berechneten Parameter/n $k$, $k_1$, $k_2$, $n$ und/oder von der Änderung des Mikroorganismen-Gehalts X infolge der Biozidzufuhr vorgeben kann. Überdies kann die Steuereinheit 11 weitere Informationen, z.B. eine Umweltverträglichkeit und/oder die Kosten jeder der Komponenten, berücksichtigen und in Abhängigkeit davon die Zusammensetzung Z vorgeben.

All diese Abhängigkeiten sind z.B. in einer Datenbank als Heuristik hinterlegt, welche zur Steuereinheit 11 gehört, damit diese heuristikbasiert eine geeignete Zusammensetzung Z vorgeben kann. Alternativ verfügt die Steuereinheit 11 optional über ein selbstlernendes System, z.B. ein neuronales Netz, insbesondere ein Long Short-Term Memory (LSTM), welches aus in der Vergangenheit vorgegebenen Zusammensetzungen Z, den daraus resultierenden Mengen P an zugeführtem Biozid B bzw. den dadurch verursachten Änderungen des Mikroorganismen-Gehalts X lernt. Anhand der jeweiligen Messwerte der Sensoren 13, 14 bzw. der Messeinheit 6 und/oder allfälliger weiterer der genannten Informationen und der Heuristik gibt die Steuereinheit 11 dann eine jeweils besonders geeignete - z.B. besonders wirksame, umweltschonende, kostengünstige - Zusammensetzung Z des Biozids B vor. Zu diesem Lernen kann das System zumindest gelegentlich zufällige Zusammensetzungen Z vorgeben.

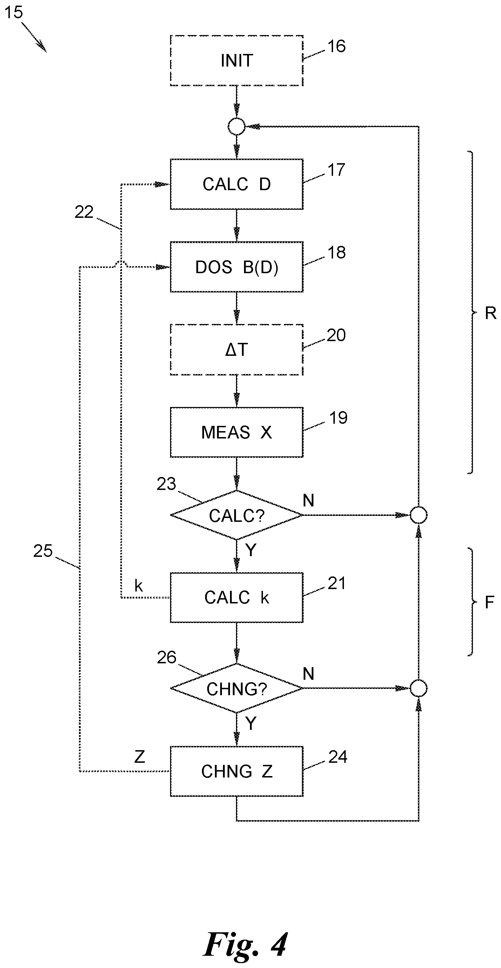

Anhand des in Fig. 4 dargestellten Beispiels werden im Folgenden verschiedene Beispiele eines von der Vorrichtung 1 ausgeführten Verfahrens 15 zum Regeln des Mikroorganismen-Gehalts X in der Flüssigkeit 2 näher erläutert.

In einem Initialisierungsschritt 16 werden z.B. der bzw. die Parameter $k$ bzw. $k_1$, $k_2$ des Modells einschließlich des Verdünnungskoeffizienten $n$ und der zu erzielende Mikroorganismen-Gehalt X* vorgegeben und wird der Mikroorganismen-Gehalt X der Flüssigkeit 2 gemessen. Darauf wird ein Regelprozess R des Verfahrens 15 entweder fortlaufend ausgeführt, z.B. mithilfe analoger Reglerbauteile, oder - wie im dargestellten Beispiel - als digitaler Regelprozess R zyklisch, insbesondere periodisch, durchlaufen. Der Regelprozess R umfasst wie zuvor beschrieben zumindest einen Schritt 17, in welchem die Regeleinheit 7 die Dosierung D des Biozids B bestimmt, einen Schritt 18, in welchem die Zuführeinheit 8 der Flüssigkeit 2 das Biozid B in der in Schritt 17 bestimmten Dosierung D zuführt und optional die Zusammensetzung Z wählt, und einen Schritt 19, in welchem die Messeinheit 6 den Mikroorganismen-Gehalt X in der Flüssigkeit 2 misst.

Wird der Regelprozess R - wie dargestellt - zyklisch durchlaufen, kann die Zuführeinheit 8 in Schritt 18 entweder unmittelbar die gesamte Dosis der von der Regeleinheit 7 bestimmten Dosierung D des Biozids B der Flüssigkeit 2 zuführen oder veranlassen, dass das Biozid B der Flüssigkeit 2 kontinuierlich, z.B. mithilfe einer Pumpe, in der bestimmten Dosierung D zudosiert wird.

Um einer lediglich langsamen Änderung des Mikroorganismen-Gehalts X nach dem Zuführen 18 von Biozid B Rechnung zu tragen, enthält optional der Regelprozess R einen Warteschritt 20, sodass beim folgenden Messen 19 bereits ein veränderter Mikroorganismen-Gehalt X zu erwarten ist.

In einem Nachführprozess F berechnet die Recheneinheit 9 in Schritt 21 den/die Parameter $k$, $k_1$, $k_2$, $n$ des Modells wie zuvor beschrieben. Mit dem/den errechneten Parameter/n $k$, $k_1$, $k_2$, $n$ des Modells wird über Pfad 22 (zu Schritt 17) die Regeleinheit 7 bzw. ihr Regler nachgeführt. Der Nachführprozess F kann parallel zum Regelprozess R oder - wie dargestellt - intermittierend dazu, d.h. den Regelprozess R jeweils unterbrechend, ausgeführt werden. In der dargestellten Variante folgt der Nachführprozess F jeweils unmittelbar auf den Regelprozess R, d.h. er unterbricht den Regelprozess R, wenn in einer Verzweigung 23 eine positive Prüfung erfolgt (Zweig "Y" der Verzweigung 23); bei negativer Prüfung (Zweig "N" der Verzweigung 23) wird der Regelprozess R weiter ausgeführt. Als Prüfkriterium wird in der Verzweigung 23 beispielsweise eine Anzahl von Durchläufen des Regelprozesses R seit dem letzten Durchlaufen des Nachführprozesses F, das Ablaufen einer Zeitspanne, z.B. des Zeitintervalls $\Delta t$, oder ein besonders hoher Wert für z.B. die von der Regeleinheit 7 bestimmte Dosierung D oder den von der Messeinheit 6 gemessenen Mikroorganismen-Gehalt X herangezogen. Alternativ kann der Nachführprozess F in jedem Zyklus unmittelbar auf den Regelprozess R folgen, d.h. das Verfahren 15 keine Verzweigung 23 haben.

In diesem Zusammenhang sei angemerkt, dass das Zeitintervall $\Delta t$ je nach Erfordernis gewählt werden kann, z.B. gleich der Periode des Regelprozesses R, wenn dieser periodisch durchlaufen wird, oder länger, insbesondere ein Vielfaches davon.

In einem optionalen Schritt 24, welcher parallel zum Regelprozess R und zum Nachführprozess F oder - wie dargestellt - intermittierend dazu ausgeführt wird, ermittelt die Steuereinheit 11 die Zusammensetzung Z des Biozids B wie zuvor beschrieben und gibt diese der Zuführeinheit 8 über Pfad 25 vor. In der dargestellten Variante folgt das Vorgeben 24 unmittelbar auf den Nachführprozess F, wenn in einer Verzweigung 26 eine positive Prüfung erfolgt (Zweig "Y" der Verzweigung 26); bei negativer Prüfung (Zweig "N" der Verzweigung 26) wird mit dem Regelprozess R fortgesetzt. Im Allgemeinen wird die Zusammensetzung Z in regelmäßigen Zeitabständen vorgegeben, d.h. dass als Prüfkriterium in der Verzweigung 26 das Erreichen des Zeitabstands vom letzten Vorgeben 24 herangezogen wird. Diese Zeitabstände sind meist wesentlich - z.B. 10-fach oder 100-fach - größer als die im Regelprozess R und im Nachführprozess F betrachteten Zeitintervalle $\Delta$t. Alternativ oder ergänzend kann als Prüfkriterium z.B. eine schwellwertunterschreitende - und somit geringer als erwartete - Abnahme des Mikroorganismen-Gehalts X trotz hoher Dosierung D oder eine Schwellwertüberschreitung des Mikroorganismen-Gehalts X oder seines Anstiegs herangezogen werden.

Die Erfindung ist nicht auf die dargestellten Ausführungsformen beschränkt, sondern umfasst alle Varianten, Modifikationen und deren Kombinationen, die in den Rahmen der angeschlossenen Ansprüche fallen.

## Patentansprüche

1. Vorrichtung zum Regeln eines Gehalts (X) an Mikroorganismen (M) in einer Flüssigkeit (2), umfassend:

   eine Messeinheit (6) zum Messen des genannten Mikroorganismen-Gehalts (X),
   eine an die Messeinheit (6) angeschlossene Regeleinheit (7), welche dazu ausgebildet ist, zur Erzielung eines vorgegebenen Mikroorganismen-Gehalts (X*) eine Dosierung (D) eines Biozids (B) anhand des gemessenen Mikroorganismen-Gehalts (X) zu bestimmen, und
   eine an die Regeleinheit (7) angeschlossene Zuführeinheit (8), welche dazu ausgebildet ist, der Flüssigkeit (2) das Biozid (B) in der von der Regeleinheit (7) bestimmten Dosierung (D) zuzuführen,
   **gekennzeichnet durch**
   eine an die Messeinheit (6) und die Regeleinheit (7) angeschlossene Recheneinheit (9),
   wobei die Regeleinheit (7) dazu ausgebildet ist, die Dosierung (D) unter Verwendung eines Modells mit zumindest einem Parameter (k; $k_1$, $k_2$; n) des Zusammenhangs zwischen einer Menge (P) an in einem Zeitintervall ($\Delta$t) zugeführtem Biozid (B) und der dadurch in diesem Zeitintervall ($\Delta$t) verursachten Änderung des Mikroorganismen-Gehalts (X) zu bestimmen, und
   wobei die Recheneinheit (9) dazu ausgebildet ist, aus einer Aufzeichnung des von der Messeinheit (6) über zumindest ein vergangenes Zeitintervall ($\Delta$t) gemessenen Mikroorganismen-Gehalts (X) und der Menge (P) an in diesem Zeitintervall zugeführtem Biozid (B) den zumindest einen Parameter (k; $k_1$, $k_2$; n) zur Verwendung durch die Regeleinheit (7) in zumindest einem späteren Zeitintervall ($\Delta$t) zu berechnen,
   wobei das genannte Modell entweder durch die Gleichung

$$\frac{dX}{dt} = -kB^n X$$

   oder durch die Gleichung

$$\frac{dX}{dt} = -k_1 B_0^n e^{-k_2 nt} X$$

   gegeben ist, mit:

   $\dfrac{dX}{dt}$ Änderung des Mikroorganismen-Gehalts (X) in einem Zeitintervall ($\Delta$t),
   k, $k_1$, $k_2$ Parameter (k; $k_1$, $k_2$) des Modells,
   B Menge (P) an in diesem Zeitintervall ($\Delta$t) zugeführtem Biozid (B),
   $B_0$ Menge (P) an in diesem Zeitintervall ($\Delta$t) zugeführtem Biozid (B) zum Zeitpunkt seiner Zufuhr,
   n Verdünnungskoeffizient (n) des Biozids (B), und
   X gemessener Mikroorganismen-Gehalt (X).

2. Vorrichtung nach Anspruch 1, **dadurch gekennzeichnet, dass** die Messeinheit (6) ein Durchflusszytometer ist, welches dazu ausgebildet ist, den Gehalt (X) an Mikroorganismen (M) einer vorgegebenen Art (A) zu messen.

3. Vorrichtung nach Anspruch 1 oder 2, **dadurch gekennzeichnet, dass** die Recheneinheit (9) dazu ausgebildet ist, den zumindest einen Parameter (k; $k_1$, $k_2$; n) nach jedem Zeitintervall ($\Delta$t) einer Folge von Zeitintervallen ($\Delta$t) aus der Aufzeichnung des von der Messeinheit (6) über zumindest ein unmittelbar vorangegangenes Zeitintervall ($\Delta$t) gemessenen Mikroorganismen-Gehalts (X) und der Menge (P) an in diesem zumindest einen unmittelbar voran-

gegangenen Zeitintervall ($\Delta t$) zugeführtem Biozid (B) zu berechnen.

4. Vorrichtung nach einem der Ansprüche 1 bis 3, **dadurch gekennzeichnet, dass** die Zuführeinheit (8) dazu ausgebildet ist, das Biozid (B) gemäß einer vorgegebenen Zusammensetzung (Z) aus einer oder mehreren Komponenten zu wählen, wobei die Vorrichtung (1) ferner eine an die Zuführeinheit (8) angeschlossene Steuereinheit (11) umfasst, welche dazu ausgebildet ist, der Zuführeinheit (8) die Zusammensetzung (Z) vorzugeben.

5. Vorrichtung nach Anspruch 4, **dadurch gekennzeichnet, dass** die Vorrichtung (1) zumindest einen Sensor (13, 14) zum Messen zumindest einer der Messgrößen pH-Wert (W), Temperatur (T), Druck und Leitfähigkeit der Flüssigkeit (2) umfasst, wobei die Steuereinheit (11) an den zumindest einen Sensor (13, 14) angeschlossen und dazu ausgebildet ist, die Zusammensetzung (Z) in Abhängigkeit von zumindest dieser/diesen Messgröße/n (W, T) vorzugeben.

6. Vorrichtung nach Anspruch 4 oder 5, **dadurch gekennzeichnet, dass** die Steuereinheit (11) dazu ausgebildet ist, in regelmäßigen Zeitabständen der Zuführeinheit (8) eine Zusammensetzung (Z) vorzugeben.

7. Verfahren zum Regeln eines Gehalts (X) an Mikroorganismen (M) in einer Flüssigkeit (2), umfassend
in einem Regelprozess (R):

Messen (19) des genannten Mikroorganismen-Gehalts (X) mithilfe einer Messeinheit (6),
Bestimmen (17) einer Dosierung (D) eines Biozids (B) in Hinblick auf die Erzielung eines vorgegebenen Mikroorganismen-Gehalts (X*) anhand des gemessenen Mikroorganismen-Gehalts (X) und eines Modells mit zumindest einem Parameter (k; $k_1$, $k_2$; n) des Zusammenhangs zwischen einer Menge (P) an in einem Zeitintervall ($\Delta t$) zugeführtem Biozid (B) und der dadurch in diesem Zeitintervall ($\Delta t$) verursachten Änderung des Mikroorganismen-Gehalts (X), und
Zuführen (18) des Biozids (B) in der bestimmten Dosierung (D) zu der Flüssigkeit (2); und
in einem zum Regelprozess (R) parallelen oder intermittierenden Nachführprozess (F):

Berechnen (21) des zumindest einen Parameters (k; $k_1$, $k_2$; n) aus einer Aufzeichnung des von der Messeinheit (6) über zumindest ein vergangenes Zeitintervall ($\Delta t$) gemessenen Mikroorganismen-Gehalts (X) und der Menge (P) an in diesem Zeitintervall ($\Delta t$) zugeführtem Biozid (B) zur Verwendung in dem Regelprozess (R) in zumindest einem späteren Zeitintervall ($\Delta t$),
wobei das genannte Modell entweder durch die Gleichung

$$\frac{dX}{dt} = -kB^n X$$

oder durch die Gleichung

$$\frac{dX}{dt} = -k_1 B_0^n e^{-k_2 nt} X$$

gegeben ist, mit:

$\dfrac{dX}{dt}$ Änderung des Mikroorganismen-Gehalts (X) in einem Zeitintervall ($\Delta t$),
k, $k_1$, $k_2$ Parameter (k; $k_1$; $k_2$) des Modells,
B Menge (P) an in diesem Zeitintervall ($\Delta t$) zugeführtem Biozid (B),
$B_0$ Menge (P) an in diesem Zeitintervall ($\Delta t$) zugeführtem Biozid (B) zum Zeitpunkt seiner Zufuhr,
n Verdünnungskoeffizient (n) des Biozids (B), und
X gemessener Mikroorganismen-Gehalt (X).

8. Verfahren nach Anspruch 7, **dadurch gekennzeichnet, dass** die Messeinheit (6) den Gehalt (X) an Mikroorganismen (M) einer vorgegebenen Art (A) misst.

9. Verfahren nach Anspruch 7 oder 8, **dadurch gekennzeichnet, dass** der Nachführprozess (F) nach jedem Zeit-

intervall (Δt) einer Folge von Zeitintervallen (Δt) ausgeführt wird, wobei der zumindest eine Parameter (k; $k_1$, $k_2$; n) aus der Aufzeichnung des von der Messeinheit (6) über zumindest ein unmittelbar vorangegangenes Zeitintervall (Δt) gemessenen Mikroorganismen-Gehalts (X) und der Menge (P) an in diesem zumindest einen unmittelbar vorangegangenen Zeitintervall (Δt) zugeführtem Biozid (B) berechnet wird.

10. Verfahren nach einem der Ansprüche 7 bis 9, **dadurch gekennzeichnet, dass** das Biozid (B) gemäß einer vorgegebenen Zusammensetzung (Z) aus einer oder mehreren Komponenten gewählt wird.

11. Verfahren nach Anspruch 10, **dadurch gekennzeichnet, dass** zumindest ein Sensor (13, 14) zumindest eine der Messgrößen pH-Wert (W), Temperatur (T), Druck und Leitfähigkeit der Flüssigkeit (2) misst und die Zusammensetzung (Z) in Abhängigkeit von zumindest dieser/diesen Messgröße/n (W, T) vorgegeben wird.

12. Verfahren nach Anspruch 10 oder 11, **dadurch gekennzeichnet, dass** in regelmäßigen Zeitabständen eine Zusammensetzung (Z) vorgeben wird.

## Claims

1. A device for regulating a content (X) of microorganisms (M) in a liquid (2), comprising:

   a measuring unit (6) for measuring said microorganism content (X),
   a regulation unit (7), which is connected to the measuring unit (6) and which is configured to determine a dosage (D) of a biocide (B) based on the measured microorganism content (X) in order to achieve a predefined microorganism content (X*), and
   a supply unit (8), which is connected to the regulation unit (7) and which is configured to supply the biocide (B) to the liquid (2) in the dosage (D) determined by the regulation unit (7),
   **characterised by**
   a computing unit (9) connected to the measuring unit (6) and the regulation unit (7),
   wherein the regulation unit (7) is configured to determine the dosage (D) using a model with at least one parameter (k; $k_1$, $k_2$; n) of the relationship between an amount (P) of biocide (B) supplied in a time interval (Δt) and the change in the microorganism content (X) caused thereby in this time interval (Δt), and
   wherein the computing unit (9) is configured to calculate the at least one parameter (k; $k_1$, $k_2$; n) for use by the regulation unit (7) in at least one later time interval (Δt) from a recording of the microorganism content (X) measured by the measuring unit (6) over at least one past time interval (Δt) and the amount (P) of biocide (B) supplied in this time interval,
   wherein said model is given either by the equation

   $$\frac{dX}{dt} = -kB^n X$$

   or by the equation

   $$\frac{dX}{dt} = -k_1 B_0^n e^{-k_2 nt} X$$

   with:

   $\frac{dX}{dt}$ change in microorganism content (X) in a time interval (Δt),
   k, $k_1$, $k_2$ parameter (k; $k_1$, $k_2$) of the model,
   B amount (P) of biocide (B) supplied in this time interval (Δt),
   $B_0$ amount (P) of biocide (B) supplied in this time interval (Δt) at the time of its supply,
   n dilution coefficient (n) of the biocide (B), and
   X measured microorganism content (X).

2. The device according to claim 1, **characterised in that** the measuring unit (6) is a flow cytometer which is configured to measure the content (X) of microorganisms (M) of a predetermined type (A).

3. The device according to claim 1 or 2, **characterised in that** the computing unit (9) is configured to calculate the at least one parameter (k; $k_1$, $k_2$; n) after each time interval ($\Delta t$) of a sequence of time intervals ($\Delta t$) from the recording of the microorganism content (X) measured by the measuring unit (6) over at least one immediately preceding time interval ($\Delta t$) and the amount (P) of biocide (B) supplied in this at least one immediately preceding time interval ($\Delta t$).

4. The device according to any one of claims 1 to 3, **characterised in that** the supply unit (8) is configured to select the biocide (B) from one or more components according to a predetermined composition (Z), wherein the device (1) further comprises a control unit (11) which is connected to the supply unit (8) and which is configured to predetermine the composition (Z) for the supply unit (8).

5. The device according to claim 4, **characterised in that** the device (1) comprises at least one sensor (13, 14) for measuring at least one of the measurands constituted by pH (W), temperature (T), pressure and conductivity of the liquid (2), wherein the control unit (11) is connected to the at least one sensor (13, 14) and is configured to predetermine the composition (Z) depending on at least this/these measurand/s (W, T).

6. The device according to claim 4 or 5, **characterised in that** the control unit (11) is configured to predetermine a composition (Z) for the supply unit (8) at regular time intervals.

7. A method for regulating a content (X) of microorganisms (M) in a liquid (2), comprising
   in a regulation process (R):

   measuring (19) said microorganism content (X) by means of a measuring unit (6),
   determining (17) a dosage (D) of a biocide (B) with a view to achieving a predefined microorganism content (X*) based on the measured microorganism content (X) and a model comprising at least one parameter (k; $k_1$, $k_2$; n) of the relationship between an amount (P) of biocide (B) supplied in a time interval ($\Delta t$) and the change in microorganism content (X) caused thereby in that time interval ($\Delta t$), and
   supplying (18) the biocide (B) in the determined dosage (D) to the liquid (2); and
   in a tracking process (F) parallel or intermittent to the regulation process (R):

   calculating (21) the at least one parameter (k; $k_1$, $k_2$; n) from a recording of the microorganism content (X) measured by the measuring unit (6) over at least one past time interval ($\Delta t$) and the amount (P) of biocide (B) supplied in that time interval ($\Delta t$) for use in the regulation process (R) in at least one later time interval ($\Delta t$), wherein said model is given either by the equation

   $$\frac{dX}{dt} = -kB^n X$$

   or by the equation

   $$\frac{dX}{dt} = -k_1 B_0^n e^{-k_2 nt} X$$

   with:

   $\dfrac{dX}{dt}$ change in microorganism content (X) in a time interval ($\Delta t$),
   k, $k_1$, $k_2$ parameter (k; $k_1$, $k_2$) of the model,
   B amount (P) of biocide (B) supplied in this time interval ($\Delta t$),
   $B_0$ amount (P) of biocide (B) supplied in this time interval ($\Delta t$) at the time of its supply,
   n dilution coefficient (n) of the biocide (B) and
   X measured microorganism content (X).

8. The method according to claim 7, **characterised in that** the measuring unit (6) measures the content (X) of microorganisms (M) of a predetermined type (A).

9. The method according to claim 7 or 8, **characterised in that** the tracking process (F) is carried out after each time interval ($\Delta$t) of a sequence of time intervals ($\Delta$t), wherein the at least one parameter (k; $k_1$, $k_2$; n) is calculated from the recording of the microorganism content (X) measured by the measuring unit (6) over at least one immediately preceding time interval ($\Delta$t) and the amount (P) of biocide (B) supplied in this at least one immediately preceding time interval ($\Delta$t).

10. The method according to any one of claims 7 to 9, **characterised in that** the biocide (B) is selected from one or more components according to a predetermined composition (Z).

11. The method according to claim 10, **characterised in that** at least one sensor (13, 14) measures at least one of the measurands constituted by pH (W), temperature (T), pressure and conductivity of the liquid (2) and the composition (Z) is predetermined depending on at least this/these measurand/s (W, T).

12. The method according to claim 10 or 11, **characterised in that** a composition (Z) is predetermined at regular time intervals.


**Revendications**

1. Dispositif de régulation d'une teneur (X) en microorganismes (M) dans un liquide (2), comprenant :

   une unité de mesure (6) pour la mesure de ladite teneur (X) en microorganismes,
   une unité de régulation (7) qui est reliée à l'unité de mesure (6) et qui est conçue pour, afin d'obtenir une teneur en microorganismes (X*) prédéfinie, déterminer un dosage (D) d'un biocide (B) à l'aide de la teneur (X) en microorganismes mesurée, et
   une unité d'alimentation (8) qui est reliée à l'unité de régulation (7), laquelle est conçue pour alimenter le biocide (B) dans le dosage (D) déterminée par l'unité de régulation (7) vers le liquide (2),
   **caractérisé par**
   une unité de calcul (9) qui est reliée à l'unité de mesure (6) et à l'unité de régulation (7),
   dans lequel l'unité de régulation (7) est conçue pour déterminer le dosage (D) moyennant l'emploi d'un modèle avec au moins un paramètre (k ; $k_1$, $k_2$ ; n) de la correspondance entre une quantité (P) en biocide (B) alimentée dans un intervalle de temps ($\Delta$t) et la modification de la teneur (X) en microorganismes produite par celle-ci dans cet intervalle de temps ($\Delta$t), et
   dans lequel l'unité de calcul (9) est conçue pour, à partir d'un enregistrement de la teneur (X) en microorganismes mesurée par l'unité de mesure (6) sur au moins un intervalle de temps ($\Delta$t) écoulé et la quantité (P) en biocide (B) alimentée pendant cet intervalle de temps, calculer l'au moins un paramètre (k ; $k_1$, $k_2$ ; n) à employer par l'unité de régulation (7) dans au moins un intervalle de temps ($\Delta$t) ultérieur,
   dans lequel ledit modèle est donné soit par l'équation

$$\frac{dX}{dt} = -kB^n X$$

   soit l'équation

$$\frac{dX}{dt} = -k_1 B_0^n e^{-k_2 nt} X$$

   avec :

   $\dfrac{dX}{dt}$ modification de la teneur (X) en microorganismes dans un intervalle de temps ($\Delta$t),
   k, $k_1$, $k_2$ paramètre (k ; $k_1$, $k_2$) du modèle,
   B quantité (P) en biocide (B) alimentée dans cet intervalle de temps ($\Delta$t),
   $B_0$ quantité (P) en biocide (B) alimentée dans cet intervalle de temps ($\Delta$t) au moment de son alimentation,
   n coefficient de dilution (n) du biocide (B) et
   X teneur (X) en microorganismes mesurée.

**2.** Dispositif selon la revendication 1, **caractérisé en ce que** l'unité de mesure (6) est un cytomètre en flux, lequel est conçu pour mesurer la teneur (X) en microorganismes (M) d'un type (A) prédéterminé.

**3.** Dispositif selon la revendication 1 ou 2, **caractérisé en ce que** l'unité de calcul (9) est conçue pour calculer l'au moins un paramètre (k ; $k_1$, $k_2$ ; n) après chaque intervalle de temps ($\Delta t$) d'une séquence d'intervalles de temps ($\Delta t$) à partir de l'enregistrement de la teneur (X) en microorganismes mesurée par l'unité de mesure (6) sur au moins un intervalle de temps ($\Delta t$) immédiat précédemment écoulé et la quantité (P) en biocide (B) alimentée dans cet au moins un intervalle de temps ($\Delta t$) immédiat précédemment écoulé.

**4.** Dispositif selon l'une des revendications 1 à 3, **caractérisé en ce que** l'unité d'alimentation (8) est conçue pour sélectionner le biocide (B) en fonction d'une composition (Z) prédéterminée à partir d'un ou de plusieurs composants, le dispositif (1) comprenant en outre une unité de commande (11) qui est reliée à l'unité d'alimentation (8) et qui est conçue pour prédéterminer la composition (Z) à l'unité d'alimentation (8).

**5.** Dispositif selon la revendication 4, **caractérisé en ce que** le dispositif (1) comprend au moins un capteur (13, 14) pour la mesure d'au moins une des grandeurs de mesure parmi la valeur de pH (W), la température (T), la pression et la conductibilité du liquide (2), où l'unité de commande (11) est reliée à l'au moins un capteur (13, 14) et est conçue pour prédéterminer la composition (Z) en fonction d'au moins cette grandeur de mesure/ces grandeurs de mesures (W, T).

**6.** Dispositif selon la revendication 4 ou 5, **caractérisé en ce que** l'unité de commande (11) est conçue pour prédéterminer à l'unité d'alimentation (8) une composition (Z) à des intervalles de temps réguliers.

**7.** Procédé de régulation d'une teneur (X) en microorganismes (M) dans un liquide (2), comprenant dans un processus de régulation (R):

la mesure (19) de ladite teneur (X) en microorganismes au moyen d'une unité de mesure (6),
la détermination (17) d'un dosage (D) d'un biocide (B) en vue de l'obtention d'une teneur en microorganismes (X*) prédéfinie à l'aide de la teneur (X) en microorganismes mesurée et d'un modèle avec au moins un paramètre (k ; $k_1$, $k_2$ ; n) de la correspondance entre une quantité (P) en biocide (B) alimentée dans un intervalle de temps ($\Delta t$) et la modification de la teneur (X) en microorganismes produite par celle-ci dans cet intervalle de temps ($\Delta t$), et l'alimentation (18) du biocide (B) dans le dosage (D) déterminée vers le liquide (2) ; et
dans un processus d'actualisation (F) parallèle ou intermittent par rapport au processus de régulation (R) :

le calcul (21) de l'au moins un paramètre (k ; $k_1$, $k_2$ ; n) à partir d'un enregistrement de la teneur (X) en microorganismes mesurée par l'unité de mesure (6) sur au moins un intervalle de temps ($\Delta t$) écoulé et la quantité (P) en biocide (B) alimentée pendant cet intervalle de temps ($\Delta t$) à employer dans le processus de régulation (R) dans au moins un intervalle de temps ($\Delta t$) ultérieur,
dans lequel ledit modèle est donné soit par l'équation

$$\frac{dX}{dt} = -kB^n X$$

soit l'équation

$$\frac{dX}{dt} = -k_1 B_0^n e^{-k_2 nt} X$$

avec :

$\dfrac{dX}{dt}$ modification de la teneur (X) en microorganismes dans un intervalle de temps ($\Delta t$),
k, $k_1$, $k_2$ paramètre (k ; $k_1$, $k_2$) du modèle,
B quantité (P) en biocide (B) alimentée dans cet intervalle de temps ($\Delta t$),
$B_0$ quantité (P) en biocide (B) alimentée dans cet intervalle de temps ($\Delta t$) au moment de son alimentation,
n coefficient de dilution (n) du biocide (B) et
X teneur (X) en microorganismes mesurée.

**8.** Procédé selon la revendication 7, **caractérisé en ce que** l'unité de mesure (6) mesure la teneur (X) en microorganismes (M) d'un type (A) prédéterminé.

**9.** Procédé selon la revendication 7 ou 8, **caractérisé en ce que** le processus d'actualisation (F) est exécuté après chaque intervalle de temps ($\Delta$t) d'une séquence d'intervalles de temps ($\Delta$t), où l'au moins un paramètre (k ; $k_1$, $k_2$ ; n) est calculé à partir de l'enregistrement de la teneur (X) en microorganismes mesurée par l'unité de mesure (6) sur au moins un intervalle de temps ($\Delta$t) immédiat précédemment écoulé et la quantité (P) en biocide (B) alimentée dans cet au moins un intervalle de temps ($\Delta$t) immédiat précédemment écoulé.

**10.** Procédé selon l'une des revendications 7 à 9, **caractérisé en ce que** le biocide (B) est sélectionné en fonction d'une composition (Z) prédéterminée à partir d'un ou de plusieurs composants.

**11.** Procédé selon la revendication 10, **caractérisé en ce que** l'au moins un capteur (13, 14) mesure au moins une des grandeurs de mesure parmi la valeur de pH (W), la température (T), la pression et la conductibilité du liquide (2) et la composition (Z) est prédéterminée en fonction d'au moins cette grandeur de mesure/ces grandeurs de mesures (W, T).

**12.** Procédé selon la revendication 10 ou 11, **caractérisé en ce que** la composition (Z) est prédéterminée à des intervalles de temps ($\Delta$t) réguliers.

*Fig. 1*

*Fig. 2*

*Fig. 3*

*Fig. 4*

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2010111639 A1
- EP 1350431 A1
- EP 3617691 A1